# FASCICULE DE BREVET EUROPEEN

(11) **EP 1 172 126 B1**
(45) Date de publication et mention de la délivrance du brevet: **06.10.2004**
(21) Numéro de dépôt: 01401852.7
(22) Date de dépôt: 11.07.2001
(51) Int. Cl.: A61N 1/375

(54) **Tête de connecteur de dispositif médical implantable actif tel que stimulteur cardiaque, défibrillateur, cardiovecteur et/ou dispositif multisite, et procédé de collage de cette tête sur le boîtier**
Kopf eines Verbinders für aktive implantierbare medizinische Vorrichtungen wie Herzschrittmacher, Defibrillator, Kardiovertierer und/oder Multisitevorrichtung und Verfahren zum Verbinden des Kopfes mit dem Gehäuse
Connector head for an active implantable medical device such as pacemaker, defibrillator, cardioverter and/or multisite device, and method for bonding the head to the case

(30) Priorité: 11.07.2000 FR 0009053
(43) Date de publication de la demande: 16.01.2002
(73) Titulaire: ELA MEDICAL, F-92541 Montrouge (FR)
(72) Inventeur: Deziz, Magid, 91140 Villebon sur Yvette (FR)
(74) Mandataire: Dupuis-Latour, Dominique

(56) Documents cités:
- EP-A- 0 408 290
- US-A- 4 853 064
- US-A- 5 683 433
- US-A- 5 871 515
- US-A- 6 009 348

## Description

L'invention concerne la fabrication des dispositifs médicaux implantables actifs. Elle sera principalement décrite dans le cas d'un stimulateur cardiaque, mais il ne s'agit là que d'un exemple de mise en oeuvre de l'invention, qui est applicable de façon beaucoup plus générale à une très grande variété de "dispositifs médicaux implantables actifs" tels que définis par la Directive 90/385/CE du 20 juin 1990 du Conseil des communautés européennes, définition qui inclut, outre les stimulateurs cardiaques, les défibrillateurs et/ou cardioverteurs, les appareils neurologiques, les pompes de diffusion de substances médicales, les implants cochléaires, les capteurs biologiques implantés, etc.

Ces dispositifs comportent un générateur contenant l'électronique du dispositif et relié électriquement et mécaniquement à une sonde, le raccordement étant réalisé par le chirurgien au moment de l'implantation.

Plus précisément, ce générateur est constitué d'un boîtier, contenant les divers circuits électroniques et la pile d'alimentation du dispositif, et d'une tête de connecteur reliée mécaniquement et électriquement au boîtier et pourvue d'un ou plusieurs logements susceptibles de recevoir la ou les sonde(s).

La fabrication du dispositif comporte généralement des étapes consistant, dans un premier temps, à réaliser le boîtier complet, dont émergent sur la face supérieure une pluralité de picots de traversée ; ces picots sont reliés aux circuits internes du boîtier et sont destinés à être reliés chacun à une borne correspondante de la tête de connecteur.

La tête de connecteur est alors montée sur le boîtier et solidarisée mécaniquement à ce dernier, en particulier par soudage des diverses bornes aux picots correspondants et/ou par d'autres techniques de solidarisation mécanique des éléments respectifs.

L'étape suivante est une étape d'enrobage consistant à noyer l'ensemble boîtier-tête de connecteur dans une résine silicone pour assurer :
- le collage de la tête de connecteur sur le boîtier, procurant ainsi une solidarisation mécanique supplémentaire entre ces deux éléments,
- l'étanchéité, entre tête et corps et autour des divers éléments tels que notamment les traversées électriques, et
- l'isolement électrique du boîtier du stimulateur sur toute ou partie de la surface externe de celui-ci, par enrobage de l'ensemble.

L'enrobage est opéré sous l'atmosphère d'azote. Un masque, par exemple une étiquette autocollante, peut être appliqué sur la partie du boîtier ne devant pas être isolée, lorsque le corps du boîtier constitue l'une des électrodes de recueil ou de stimulation.

Cette technique de montage est une méthode éprouvée et économique, mais elle présente notamment l'inconvénient d'un temps de fabrication important, car il est nécessaire d'attendre le séchage complet de la colle avant de pouvoir passer à la suite des opérations.

Plus précisément, les étapes de collage et enrobage mettent généralement en oeuvre une résine de type silicone, par exemple d'un type commercialisé par Rehau GmbH, impliquant une réticulation pendant une durée typique de 14 heures.

L'invention propose de modifier le procédé existant de manière à réduire considérablement - typiquement à une durée de quelques minutes - le temps nécessaire pour l'exécution des étapes de collage de la tête de connecteur sur le boîtier et/ou d'enrobage du boîtier. Un procédé qui vise aussi à réduire le temps d'assemblage est decrit dans le document US-A-5871515.

Essentiellement, l'invention propose d'utiliser, au lieu d'une résine silicone traditionnelle, une résine photoréticulable, notamment réticulable aux UV, et de modifier en conséquence la manière dont sont exécutées les étapes correspondantes de collage et/ou d'enrobage dans le processus de fabrication.

Plus précisément, l'invention propose une tête de connecteur comportant : des moyens d'assemblage mécanique et électrique sur un boîtier du dispositif ; une lèvre périphérique dont la surface en vis-à-vis du boîtier épouse ce dernier de façon étanche, de manière à définir à l'interface entre boîtier et tête de connecteur un volume fermé apte à recevoir une quantité de colle photodurcissable ; un orifice d'injection de ladite colle, communiquant avec ledit volume fermé ; et au moins un orifice d'évent communiquant également avec ce volume fermé. Cette tête est réalisée en un matériau transparent ou translucide permettant l'irradiation de ladite quantité de colle par une lumière de longueur d'onde adaptée au durcissement de la colle. Avantageusement, l'orifice d'injection de colle est situé à l'une des extrémités du volume fermé et le ou les orifice(s) d'évent sont situés à l'extrémité opposée de ce même volume.

L'invention couvre également un procédé de montage caractérisé par les étapes suivantes : a) obtention d'une tête de connecteur comportant une surface définissant une interface après liaison de la tête au boîtier ; b) assemblage mécanique et électrique de la tête de connecteur sur le boîtier ; c) injection d'une quantité de colle photodurcissable, notamment d'une résine silicone photoréticulable, à l'interface entre le boîtier et la tête de connecteur ; d) irradiation, notamment essentiellement dirigée dans le plan de l'interface entre le boîtier et la tête de connecteur, de cette quantité de colle par une lumière de longueur d'onde adaptée au durcissement de la colle ; e) apposition éventuelle d'un masque sur une partie du boîtier ; f) immersion du boîtier muni de sa tête de connecteur dans une résine photodurcissable, notamment une résine silicone photoréticulable, de manière à former sur le boîtier et la tête de connecteur une couche de revêtement de résine photodurcissable et ; g) irradiation de cette couche de résine par une lumière de longueur d'onde adaptée au durcissement de la résine.

On va maintenant décrire un exemple de mise en oeuvre de l'invention, en référence aux dessins annexés.

La figure 1 est une vue perspective, de dessus, d'un ensemble boîtier-tête de connecteur assemblé par collage selon l'invention.

La figure 2 est une vue perspective, de dessous, de la tête de connecteur, où l'on peut voir de façon plus précise la configuration de la région destinée à venir en contact avec le boîtier et à être collée sur celui-ci.

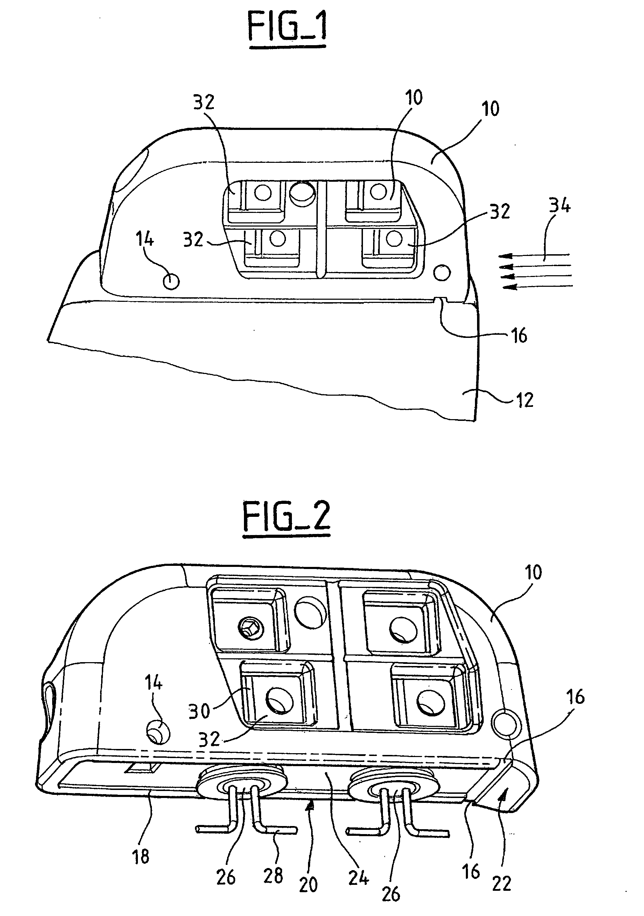

Sur la figure 1, on a illustré une tête de connecteur 10 montée sur un boîtier 12 tel qu'un boîtier de stimulateur cardiaque.

La tête 10 est une tête en matière synthétique isolante et translucide, qui peut être souple ou rigide. Le matériau de cette tête de connecteur est habituellement un silicone, dans le cas d'une tête souple, ou un dérivé de polyuréthanne tel que le *Tecothane*, dans le cas d'une tête rigide.

Le boîtier du stimulateur, quant à lui, est généralement en titane.

Selon l'invention, on utilise pour coller la tête de connecteur au boîtier une colle silicone photoréticulable, notamment une colle réticulable aux UV telle que la colle *1138* commercialisée par Dymax, USA.

Une fois la tête de connecteur 10 solidarisée mécaniquement et électriquement au boîtier 12, la colle est injectée à l'interface boîtier-tête de connecteur, par exemple au moyen d'une seringue dont l'aiguille est introduite dans un orifice d'injection 14. Cet orifice d'injection 14 est situé au voisinage de l'une des extrémités de la tête, de sorte que l'opérateur peut suivre l'avancée progressive du front de colle le long de l'interface, la tête étant en un matériau transparent ou translucide.

L'injection est poursuivie jusqu'à ce que le front de colle soit parvenu à l'autre extrémité de la tête, ce qui peut être détecté par la sortie de la colle par un ou plusieurs orifices d'évent 16 situés à l'extrémité de la tête opposée à celle de l'orifice d'injection 14. Le surplus de colle sortant des orifices 16 sera ensuite essuyé.

Plus précisément, comme on peut le voir figure 2, l'espace recevant la colle est délimité par un rebord périphérique 18 dont la face 20, 22 en contact avec le boîtier du stimulateur épouse la surface de ce dernier, de manière à constituer un volume fermé ne communiquant avec l'extérieur que par l'orifice d'injection 14 et les orifices d'évent 16. Le volume 24 va ainsi se trouver rempli de colle, la colle contournant par ailleurs les inserts 26 faisant saillie du boîtier, en assurant ainsi l'étanchéité autour de ces éléments.

Les inserts 26 portent des picots 28 débouchant en 30 dans des cavités correspondantes 32 de la tête de connecteur 10. Ces cavités 32 recevront ultérieurement les bornes de connexion et l'ensemble sera fermé par collage final d'un couvercle de fermeture (non représenté).

Une fois la colle injectée dans le volume 24 de la manière indiquée plus haut, la tête de connecteur (qui est en matériau transparent ou translucide) est irradiée par une source lumineuse dont la longueur d'onde correspond à celle permettant la réticulation de la colle choisie.

De préférence, comme schématisé en 34, le faisceau lumineux est dirigé approximativement selon le plan de l'interface boîtier-tête pour permettre une meilleure irradiation du volume 24 contenant la colle ; cette irradiation est bien entendu rendue possible par la nature transparente ou translucide du matériau de la tête.

La réticulation est obtenue en un temps très court, de quelques minutes au plus au lieu de plusieurs heures (typiquement 14 heures) comme cela était le cas avec les colles silicone traditionnelles utilisées jusqu'à présent L'enrobage, quant à lui, est effectué avec une résine silicone photoréticulable de même type que celle utilisée pour le collage de la tête de connecteur. Les différentes manipulations de l'étape d'enrobage sont en elle-même connues et ont été décrites au début de la présente description.

Compte tenu des diverses opérations annexes précédant et suivant le collage et l'enrobage, la durée d'un cycle complet de fabrication d'un stimulateur peut être ainsi raccourci de deux à trois jours, avec bien entendu tous les avantages tant économiques que techniques qui en découlent.

On notera que la technique de l'invention peut être transposée à la fabrication d'autres éléments de dispositifs implantables, notamment pour le collage des différents éléments des sonde de stimulation.

## Revendications

1. Une tête de connecteur (10) de dispositif médical implantable actif tel que stimulateur cardiaque, défibrillateur, cardioverteur et/ou dispositif multisite, **caractérisée en ce qu'**elle comporte :
- des moyens d'assemblage mécanique et électrique sur un boîtier (12) du dispositif,
- une lèvre périphérique (18) dont la surface (20, 22) en vis-à-vis du boîtier épouse ce dernier de façon étanche, de manière à définir à l'interface entre boîtier et tête de connecteur un volume fermé (24) apte à recevoir une quantité de colle photodurcissable,
- un orifice d'injection de ladite colle (14), communiquant avec ledit volume fermé (24), et
- au moins un orifice d'évent (16) communiquant également avec ce volume fermé (24),
et **en ce qu'**elle est réalisée en un matériau transparent ou translucide permettant l'irradiation de ladite quantité de colle par une lumière de longueur d'onde adaptée au durcissement de la colle.

2. La tête de connecteur de la revendication 1, dans laquelle l'orifice d'injection de colle (14) est situé à l'une des extrémités du volume fermé (24) et le ou les orifice(s) d'évent (16) sont situés à l'extrémité opposée de ce même volume (24).

3. Un procédé de montage d'un dispositif médical implantable actif tel que stimulateur cardiaque, défibrillateur, cardioverteur et/ou dispositif multisite comprenant une tête de connecteur (10) et un boîtier (12),
procédé **caractérisé par** la succession des étapes suivantes :
a) obtention d'une tête de connecteur comportant une surface définissant une interface après liaison de la tête au boîtier,
b) assemblage mécanique et électrique de la tête de connecteur sur le boîtier,
c) injection d'une quantité de colle photodurcissable à ladite interface entre le boîtier et la tête de connecteur,
d) irradiation de cette quantité de colle par une lumière de longueur d'onde adaptée au durcissement de la colle,
e) apposition éventuelle d'un masque sur une partie du boîtier,
f) immersion du boîtier muni de sa tête de connecteur dans une résine photodurcissable, de manière à former sur le boîtier et la tête de connecteur une couche de revêtement de résine photodurcissable, et
g) irradiation de cette couche de résine par une lumière de longueur d'onde adaptée au durcissement de la résine.

4. Le procédé de la revendication 3, dans lequel l'étape c) est une étape d'injection d'une résine silicone photoréticulable.

5. Le procédé de la revendication 3, dans lequel l'étape d) est une étape d'irradiation essentiellement dirigée dans le plan de l'interface entre le boîtier et la tête de connecteur.

6. Le procédé de la revendication 3, dans lequel l'étape f) est une étape d'immersion dans une résine silicone photoréticulable.

## Patentansprüche

1. Kopf eines Verbinders (10) einer aktiven implantierbaren medizinischen Vorrichtung, wie eines Herzschrittmachers, Defibrilators, Cardioverters und/oder einer Multisite-Vorrichtung, **dadurch gekennzeichnet, dass** er umfasst:
- Mittel zum mechanischen und elektrischen Zusammenbau auf einem Gehäuse (12) der Vorrichtung,
- eine umlaufende Lippe (18), deren Oberfläche (20, 22) gegenüber dem Gehäuse sich gegenüber dem letzteren in dichter Weise anpasst, um an der Schnittstelle zwischen dem Gehäuse und dem Kopf des Verbinders ein geschlossenes Volumen (24) auszubilden, das geeignet ist, eine Menge von lichthärtbarem Kleber aufzunehmen,
- eine Öffnung zum Einspritzen des Klebers (14), welche mit dem geschlossenen Volumen (24) kommuniziert, und
- mindestens eine Abzugsöffnung (16), die ebenfalls mit dem geschlossenen Volumen (24) kommuniziert,
und dadurch, dass er aus einem transparenten oder transluciden Material gebildet ist, welches die Bestrahlung der Menge von Kleber durch ein Licht ermöglicht, mit einer Wellenlänge, die dem Härten des Klebers angepasst ist.

2. Kopf eines Verbinders nach Anspruch 1, in welchem die Öffnung zum Einspritzen des Klebers (14) an einem der Enden des geschlossenen Volumens (24) liegt und die Abzugsöffnung(en) (16) am gegenüberliegenden Ende desselben Volumens (24) liegen.

3. Verfahren zur Montage einer aktiven implantierbaren medizinischen Vorrichtung, wie eines Herzschrittmachers, Defibrilators, Cardioverters und/oder von Multisite-Vorrichtungen, umfassend einen Kopf eines Verbinders (10) und ein Gehäuse (12),
wobei das Verfahren durch die Abfolge der folgenden Schritte gekennzeichnet ist:
a) Härten eines Kopfes eines Verbinders, der eine Oberfläche aufweist, die eine Schnittstelle mit Verbindung des Kopfes zum Gehäuse definiert,
b) mechanisches und elektrisches Zusammensetzen des Kopfes des Verbinders auf dem Gehäuse,
c) Einspritzen einer Menge von lichthärtbarem Kleber in die Schnittstelle zwischen dem Gehäuse und dem Kopf des Verbinders,
d) Bestrahlen dieser Menge von Kleber durch ein Licht mit einer Wellenlänge, die an die Härtung des Klebers angepasst ist,
e) eventuelles Anbringen einer Maske auf einem Teil des Gehäuses,
f) Eintauchen des mit einem Kopf eines Verbinders ausgestatteten Gehäuses in ein lichthärtbares Harz, um auf dem Gehäuse und dem Kopf eines Verbinders einen Überzug aus lichthärtbarem Harz zu bilden, und
g) Bestrahlen dieser Harzschicht durch ein Licht mit einer Wellenlänge, die an das Härten des Harzes angepasst ist.

4. Verfahren nach Anspruch 3, in welchem Schritt c) ein Schritt zum Einspritzen eines fotovernetzten Silikonharzes ist.

5. Verfahren nach Anspruch 3, in welchem Schritt d) ein Schritt des Bestrahlens ist, der im Wesentlichen in die Ebene der Schnittstelle zwischen dem Gehäuse und dem Kopf des Verbinders gerichtet ist.

6. Vorrichtung nach Anspruch 3, in welchem Schritt f) ein Schritt des Eintauchens in ein fotovemetztes Silikonharz ist.

## Claims

1. A connector head (10) for an active implantable medical device such as a cardiac pacemaker, defibrillator, cardioverter and/or multisite device, **characterised in that** it comprises:
- means for mechanically and electrically assembling said head onto a case (12) of the device,
- a peripheral lip (18) which surface (20, 22) facing the case mates the latter in a tight manner, so as to define at the interface between the case and the connector head a closed volume (24) adapted to receive a quantity of a photo-hardening adhesive,
- an opening for injecting said adhesive (14), communicating with said closed volume (24), and
- at least one vent hole (16) also communicating with said closed volume (24),
and **in that** it is made from a transparent or translucent material enabling the irradiation of said adhesive quantity by a light which wavelength is adapted to the hardening of the adhesive.

2. The connector head of claim 1, wherein said adhesive injection opening (14) is located at one end of the closed volume (24) and the vent hole(s) (16) are is located at the opposite end of said volume (24).

3. A process for assembling an active implantable medical device such as a cardiac pacemaker, defibrillator, cardioverter and/or multisite device comprising a connector head (10) and a case (12),
said process being **characterised by** the following sequence of steps:
a) providing a connector head including a surface defining an interface after the head being connected to the case,
b) mechanically and electrically assembling the connector head onto the case,
c) injecting a quantity of a photo-hardening adhesive into said interface between the case and the connector head,
d) irradiating said quantity of adhesive by a light which wavelength is adapted to the hardening of the adhesive,
e) optionally affixing a mask on a portion of the case,
f) immersing the case equipped with its connector head unit into a photo-hardening resin, so as to form on the case and the connector head a covering layer of photo-hardening resin coating, and
g) irradiating said resin layer with a light which wavelength is adapted to the hardening of the adhesive.

4. The process of claim 3, wherein step c) is a step of injection of a photo-crosslinking silicone resin.

5. The process of claim 3, wherein step d) is a step of irradiation primarily directed in the plane of the interface between the case and the connector head.

6. The process of claim 3, wherein step f) is a step of immersion in a photo-crosslinking silicone resin.
